# EUROPEAN PATENT SPECIFICATION

(11) **EP 0 440 229 B1**
(45) Date of publication and mention of the grant of the patent: **20.03.1996**
(21) Application number: 91101291.2
(22) Date of filing: 31.01.1991
(51) Int. Cl.: C07C 211/63, C07C 209/04, C07C 67/10, C07D 233/12, C11D 3/20, D06M 13/46

(54) **Process and composition for multicomponent 100% solid fabric softeners**
Verfahren zur Herstellung und Zusammensetzung von mehrkomponentigen hundertprozent festen Wäscheweichmachmitteln
Composition et procédé de fabrication d'adoucisseurs textiles solides à cent pour cent constitués par plusieurs composants

(30) Priority: 31.01.1990 US 472738
(43) Date of publication of application: 07.08.1991
(73) Proprietor: WITCO CORPORATION, Greenwich, Connecticut (US)
(72) Inventor: Ackermann, Jeannene A., Upper Arlington, Ohio (US); Miller, Michael, Columbus, Ohio (US); Whittlinger, David E., Janesville, Wisconsin (US)
(74) Representative: Schmidt-Evers, Jürgen, Dipl.-Ing.

(56) References cited:
- EP-A- 0 048 163
- EP-A- 0 345 475
- US-A- 4 237 064
- US-A- 4 851 141

## Description

The invention relates to a method of producing a mixture of a quaternary ammonium compound, fatty acid, fatty acid ester and tertiary amine salt in situ which is a highly functional mixture that is manufactured in a single step reaction whereby quaternization is completed without the aid of solvents, especially flammable solvents. The single step reaction process eliminates separate blending of individual components.

The preparation of quaternary ammonium compounds is usually conducted in stainless steel or glass-lined equipment to which a tertiary amine is charged and a solvent. Flammable solvents such as isopropyl alcohol are generally used although mixtures of isopropyl alcohol and water sometimes are employed or water alone is used. Flammable solvents are also undesirable because they are a fire hazard and special handling procedures are required when they are used. In many applications, these solvents have to be stripped from the mixture when the reaction is completed because the ultimate use of the product is in a solventless or solid form.

After the reactants are loaded into the reactor they are heated to a temperature of about 50 to about 100°C after which a quaternizing reagent is added. In some instances an exotherm is produced as a result of the quaternizing reaction and the reactor and its contents have to be cooled. The rate of addition of the quaternizing agent can also be controlled in order to minimize or eliminate the exotherm. As noted previously, in some instances, the solvent, if any is employed in the quaternizing reaction is stripped from the quaternary ammonium compound obtained since some commercial uses for the compounds are in solventless systems. Additionally, bulk shipments of quaternary ammonium compounds with solvents adds to transportion costs which is another reason to remove the solvents.

The largest use for quaternary ammonium compounds is as a fabric softener and presently accounts for more than about three quarters of the total market for these material. Some fabric softeners are supplied as a liquid dispersion of from about 3% to about 10% by weight of the quaternary ammonium compound which is adapted to be added during the rinse cycle of a commercial or home laundering operation. Another significant fabric softening application is the utilization of quaternary ammonium compounds in combination with a substrate such as a nonwoven fabric or a polymeric foam such as a polyurethane foam, this substrate so treated being added to a fabric dryer such as a clothes dryer while the fabric or clothes are still damp. The quaternary ammonium compound is formulated usually with a fatty acid ester which promotes the transfer of the quaternary ammonium compound from the nonwoven or porous polymeric substrate to the fabric or clothes.

Lastly, quaternary ammonium compounds are now being added to both solid and liquid laundry detergent compositions so that the quaternary ammonium compound can be incorporated as a fabric softener during the wash cycle of fabrics or clothes. The most successfully utilized quaternary ammonium compounds in this last respect are the dimethyl (dihydrogenated tallow) ammonium chlorides or methyl sulfates. Other quaternary ammonium compounds such as imidazolines and amidoamine quaternaries are also used in this regard.

Where the quaternary ammonium compound is used on a substrate for transfer to fabric or clothing in a fabric or clothes dryer, it has generally been the practice to separately blend the fatty acid ester into the quaternary ammonium compound which involves the extra manufacturing steps of transportion, handling and blending of the fatty acid esters into the quaternary ammonium compounds. If these steps can be eliminated a cost savings could be realized.

Quaternary ammonium compounds are also used to manufacture organomodified clays which may be added to drilling muds utilized in drilling oil wells, the organomodified clay providing improved lubrication and rheological properties of the drilling muds. These organoclays are also employed as thixotropic agents in plastisols, organosols, paints and other protective coatings, grease additives, foundry additives, cosmetics, resins and printing inks. The most common quaternary ammonium compounds employed in this regard are methyldi(hydrogenated tallow) benzylammonium chloride, dimethyldi(hydrogenated tallow) ammonium chloride and dimethyl(hydrogenated tallow) benzylammonium chloride.

Quaternary ammonium compounds are also employed as bactericides the most common of which is the quaternary ammonium compound of benzylchloride and a dimethylalkylamine, the alkyl group having from about 12 to about 16 carbon atoms as well as trimethyl alkyl ammonium chlorides where the alkyl group is a long chain alkyl such as an octadecyl group. Additionally, dimethyldicoconut-oil fatty ammonium chlorides are also effective disinfectants e.g. bactericides or bacteristats, especially against anaerobic bacteria which are sulfate reducers that are found in oil wells, these bacteria causing severe corrosion problems and plugging of formations which this type of quaternary ammonium compound can minimize or eliminate. Additionally, these quaternary ammonium compounds effective against anaerobic bacteria are also effective in removing oil from sand stone formations in oil wells and provide a two-fold effect of functioning not only as a bactericide but also in promoting so-called secondary recovery of oil.

An additional use of quaternary compounds is in hair treatment because of the antistatic effects obtained with such compounds, as well as the increased wetting which promotes improvements in both wet and dry combing or brushing and improves luster and feel. The most commonly used guaternary ammonium compounds in this respect are trimethylalkylammonium chloride, pentaethoxystearylammonium chloride, dimethylstearylbenzylammonium chloride and dimethyldialkylammonium chlorides.

The present invention relates to a method of producing a mixture of quaternary ammonium compound, fatty acid, fatty acid ester and tertiary amine salt by single-step reacting in a solvent free system, an amine, quaternizing agent, and fatty acid each in an amount so that said mixture will contain at least a quaternary ammonium compound, fatty acid, fatty acid ester and tertiary amine salt in amounts as defined in claim 1.

The present invention also includes as a composition of matter, a solvent free mixture with defined amounts of
a quarternary ammonium compound having the formula: 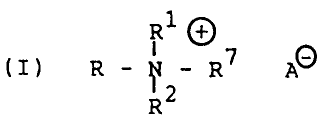
or 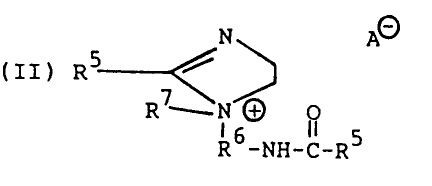
where R, R¹ and R² are
(1) linear or branched chain aliphatic saturated or unsaturated hydrocarbon group having up to about 22 carbon atoms;
(2) a hydroxy lower alkyl group;
(3) an alkyl amido alkylene group of the formula, 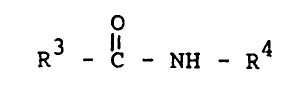 where R⁴ is lower alkylene and R³ is (1), (2), (4) or (5);
(4) a lower alkoxy group;
(5) a poly(oxyloweralkylene) group;
so that at least one of R, R¹ or R² is one of said hydrocarbon groups or one of said hydroxy lower alkyl groups
where R⁵ is a linear or branched chain aliphatic saturated or unsaturated hydrocarbon group having up to about 22 carbon atoms and R⁶ is a lower alkylene group,
where A⁻ is an anion;
a linear or branched chain saturated or unsaturated fatty acid having from 12 to 22 carbon atoms;
a tertiary amine salt having formula (I) or (II) where R⁷ is hydrogen
and a fatty acid ester having the formula: 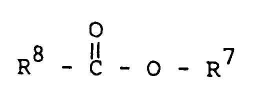
wherein R⁷ is a lower alkyl group or cyclo lower alkyl group and R⁸ is a linear or branched chain, aliphatic saturated or unsaturated group having from 11 to 21 carbon atoms.

It has been found that a highly functional mixture of a quaternary ammonium compound, a fatty acid ester, an ammonium salt and a fatty acid can be obtained in a single step reaction without the aid of solvents, especially flammable solvents to thereby produce a mixture of highly functional components. The single step reaction thereby eliminates the separate blending of such individual components as the fatty acid ester, the ammonium salt and the fatty acid with the quaternary ammonium compound.

The EP-A-0345475 describes a method of producing quaternary ammonium salts of long-chain, aliphatic carboxylic acids; this method can also be carried out without a solvent (cf. page 4, line 52 et seqq.). According to EP-A-0345475 a conversion of the various partial components in steps is expressly provided (see e.g. Example 3) and also a compound corresponding to the formula R⁴ OCOOCH₃ (this being dimethylcarbonate or the methyl/ethyl mixed ester of carbonic acid) is necessarily used as a reaction partner (II) - see e.g. claim 1 or page 2, lines 52/53. The use of such a compound is not provided in the claimed method, however. Moreover, the reaction mixture itself according to this document contains three components (see e.g. cited Example 3), namely the quaternary ammonium compound together with the amine salt and the fatty acid in quantities totalling 100 wt. %. In the present case, however, it is proceeded such that a fatty acid ester is also present in the resultant mixture of the one-stage reaction.

The advantage of the method of the present invention is that it provides a composition that is non-flammable and it is ready to be flaked or powdered because of its bulk form and can be used as a fabric softener, a fabric lubricant, a hair condition as well as an anti-static agent that can be released from a substrate such as a nonwoven fabric or a polymeric foam. These mixtures can also be used in combination with synthetic detergents or as lubricant, an emulsifier, in cosmetic preparations hair conditioning components clay modifiers, bactericidal compositions and as a floculant.

As noted, the invention relates to a method for producing a quaternary ammonium mixture that is highly functional by means of a single step reaction as well as the various compositions that can be obtained thereby and therewith.

In its broadest aspect the invention is directed to a method of producing in a single step reaction a mixture of a quaternary ammonium compound, fatty acid, fatty acid ester and tertiary amine salt by reacting a tertiary amine, quaternizing agent and fatty acid, each in amount so that said mixture will contain at least a quaternary ammonium compound, a fatty acid, fatty acid ester and a tertiary amine salt in percentages as defined in claim 1. The tertiary amine employed according to the invention has the formula: 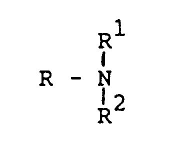
where R, R¹ and R² can be any of the following in any combination.
(1) linear or branched chain saturated or unsaturated hydrocarbon groups having up to 22 carbon atoms;
(2) a lower hydroxy alkyl group;
(3) an alkyl amido alkylene group of the formula: 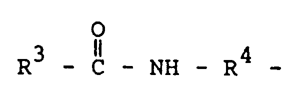 where R⁴ is lower alkylene and R³ is (1), (2), (4) or (5);
(4) lower alkoxy group;
(5) poly(oxyloweralkylene) group;
so that at least one of R, R¹ or R² is one of said linear or branched chain aliphatic saturated or unsaturated hydrocarbon groups;
or said tertiary amine is an imidazoline of the formula: 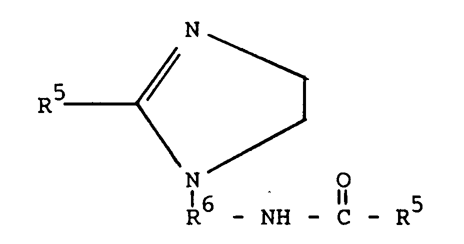
where R⁵ is a linear or branched chain, aliphatic saturated or unsaturated hydrocarbon group having up to 22 carbon atoms and R⁶ is a lower alkylene group.

Said quaternizing agent is known in the art and produces a quaternary ammonium compound having an anion A⁻. Generally these quaternizing agents are those having the formula R⁷₍ₐ₎X;
where R⁷ is a lower alkyl group or cyclo lower alkyl group such as benzyl, cyclohexylmethyl, tolyl, xylyl, naphthylmethyl, and X may be a chlorine, iodine, bromine, sulfate, methyl sulfate, carbonate, phosphate, borate group, formate, acetate, propionate, adipate, benzoate and the like and (a) is the valence of X.

Said fatty acid is a linear or branched chain aliphatic saturated or unsaturated fatty acid having from 12 to 22 carbon atoms based on coconut oil, vegetable oils, seed oils animal fats and fish oils.

Various tertiary amines that can be employed in this regard include:
dimethylamino propyl amine, amino ethylanolamine,
distearly methyl amine,
dihydrogenated tallow methyl amine,
ditallow methyl amine,
dimethyl hydrogenated tallow amine,
dimethyl coco amine,
distearyl ethoxyethyl amine,
stearyl bis-hydroxyethyl amine,
stearyl bis (polyethoxy ethanol) amine,
bis (tallowamidoethyl) 2-hydroxyethyl amine,
bis (tallowamidoethyl) 2-hydroxylpropyl amine,
1-hydrogenated tallow amido ethyl -2-hydrogenated tallow imidazoline
1-ethylene bis (2tallow, 1-methyl, imidazolinium) dimethyl amino propyl tallow amido-amine and
hydrogenated tallow hydroxyethyl imidazoline 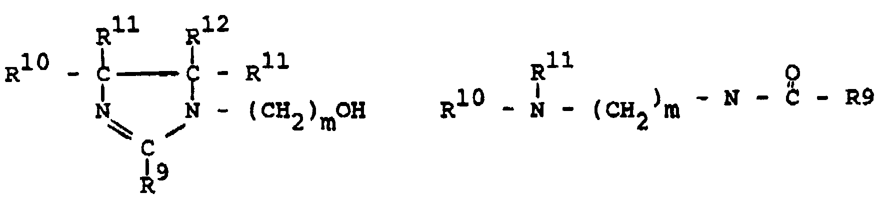
where R⁹, R¹⁰, R¹¹ and R¹² are subsequently defined.

The prior art also disclosed other amines that may be used such as those described in the United States Patents to Marschner, U.S. 4,859,456; Caswell et al., U.S. 4,857,213; Demangeon, U.S. 4,851,141 and Mermelstein et al., U.S. 4,844,824 (also referred to hereafter as "the prior art cited herein.")

Imidazoline compounds that may be used are also described in Demangeon (supra), whereas the quarternizing agents include;
dimethyl sulfate,
diethyl sulfate,
methyl chloride,
methyl bromide and
benzyl chloride as well as those cited in the prior art cited herein.

The quaternary ammonium compound thus obtained has the formula: 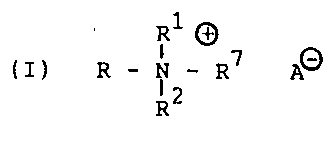
or 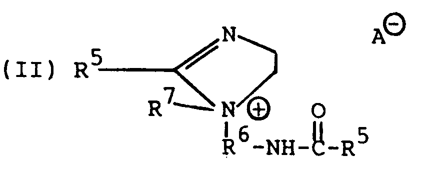
where R, R¹, R², R³, R⁴, R⁵, R⁶ and R⁷ have been defined above;
where A⁻ is an anion based on R⁷₍ₐ₎X or equivalent anions known in the quaternary ammonium compound art; 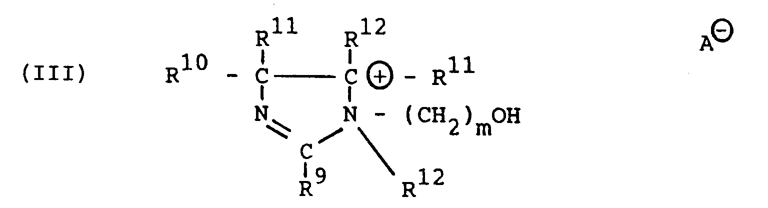
where R⁹ is C8 to C30 preferably C12 to C18 alkyl or akenyls
R¹⁰ and R¹¹ are independently hydrogen or C1 to C4 alkyl R¹² is C1 to C4 alkyl
m is 2 or 3
A⁻ is preferably an anion such as chloride, bromide, sulfate, methosulfate, nitrite, phosphate and carboxylate (i.e., acetate, adipate, propionate, benzoate) 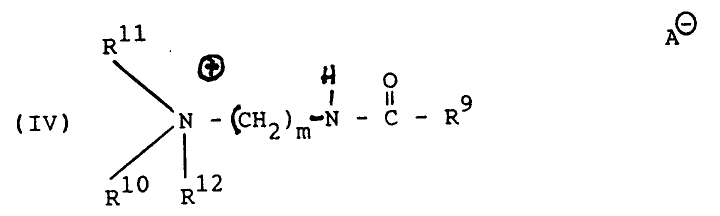
where R¹⁰, R¹¹, R¹², m and A⁻ have been previously defined and R⁹ is an alkyl or alkenyl having 8 to 22 carbon atoms.

The tertiary amine salt has the formula: 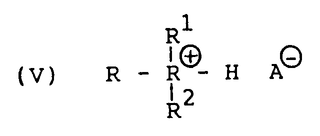
or 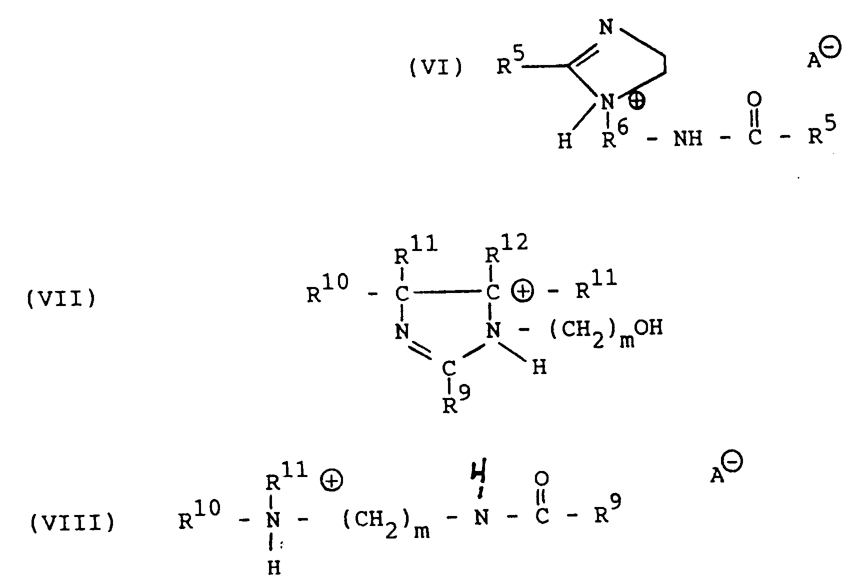
The fatty acid ester has the formula: 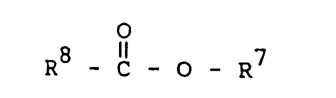
where R⁷ is a lower alkyl group or cyclo lower alkyl group and R⁸ is a linear or a branched chain aliphatic saturated or unsaturated hydrocarbon group having from 11 to 21 carbon atoms.

As used throughout this specification the terms lower alkoxy, lower alkylene and lower alkyl are intended to include compounds having up to 4 carbon atoms including the various isomeric configurations thereof e.g. t-butyl, i-butyl, i-propyl and the like and the various mixtures thereof whether such mixtures of such groups contain components having one, or two, or three or four carbon atoms and also where such groups individually or in combination are in any of their isomeric forms.

According to the method of the invention the tertiary amine, quaternizing agent and fatty acid are reacted in a single step reaction with one another each in an amount so that the mixture obtained contains from 10 weight percent to 80 weight percent and especially from about 35 weight percent to about 55 weight percent of said quaternary ammonium compound, 15 weight percent to 70 weight percent and especially from about 10 weight percent to about 30 weight percent of said salt, 1 weight percent to 70 weight percent and especially from about 5 weight percent to about 25 weight percent of said ester and from 5 weight percent to 70 weight percent and especially from about 15 weight percent to about 35 weight percent of said fatty acid wherein the ratio of said quaternary ammonium compound to said salt is from about 2:1 to about 1:2.

In one embodiment, the method of the invention is preferably practiced so that the quaternary ammonium compound that is obtained has the structural formula (I) wherein at least one of R, R¹ and R² is a branched chain or linear aliphatic saturated or unsaturated hydrocarbon group having from 12 to 22 carbon atoms, preferably saturated, and especially those based on hard tallow acids (i.e. hydrogenated tallow fatty acids) and the balance, if any of the aforesaid R, R¹ and R² groups is a lower alkyl group and R⁷ is a lower alkyl group. In another embodiment X is a sulfate group.

The invention also relates to a mixture of the various quaternary ammonium compounds as described above in combination with the various fatty acid esters, fatty acids, and tertiary amine salts also as described previously and in the amounts and ratios as described previously.

The invention also relates to a fabric softening article of manufacture comprising a fabric softening amount of any of the mixtures as described herein operatively associated with a substrate that will release such mixture to a fabric under fabric softening conditions encountered in a fabric or clothes dryer and includes the use of any of the aforesaid mixtures in a fabric softening relationship with a nonwoven or woven fiber or a polymeric open-celled or substantially open-celled foam substrate, such combination being prepared in a manner well known in the art. The mixture is employed in an amount from about 0.1 to about 10 gms and especially 1 to about 39 grns per 9 in. by 11 in. (0,065 to ≈ 2.5 g per 22,86 cm X 27,94 cm) sheet.

The various polymeric foams that are employed in this respect comprise polyurethane foams as well as any of the art known equivalent foams.

Additionally, the invention is directed to a fabric cleaning composition comprising a detergent in combination with a fabric softening amount of any of the mixtures described herein. These detergents can be any of the art known anionic, cationic, nonionic or amphoteric synthetic detergents or wetting agents that are well known in the art or a soap i.e. the reaction product of a fatty acid with a alkaline hydroxide that is water soluble e.g. fatty acid reaction products of sodium, potassium or ammonium hydroxides or amines or the art known equivalents thereof.

These surfactants are further described in Kirk-Othmer, Encyclopedia of Chemical Technology, 3rd Edition Vol. 22 pp. 332-432 which is incorporated herein by reference. Some specific detergents that are especially suitable in this regard include:
Detergents that can be employed in this respect comprise:
alkyl benzene sulfate,
sodium lauryl ether sulfate
nonyl phenyl ethoxylates and
alkyl alcohol ethoxylates as well as those noted in the prior art cited herein.

The mixture of the invention is incorporated into the detergents (whether solid or liquid) by blending in a manner well known in the art. The amount of the mixture employed is any where from about 1 to about 50 and especially from about 2 to about 30 wt. % based on the quaternary ammonium compound and the active components of the detergent i.e. the component of the detergent that has both organophilic and hydrophilic groups.

The following example is illustrative.

The method of the invention as well as a mixture obtained according to this method is exemplified by the preparation of a mixture employing the following components in the indicated amounts.

| Components | M.W. | Moles | % by Wt. |
|---|---|---|---|
| Dihydrogenated Tallow-Methyl-Amine | 523 | 1.00 | 56.63 |
| Stearic Acid | | | 30.00 |
| Dimethyl Sulfate | 126 | 0.98 | 13.37 |

The amine was charged to a clean, dry reactor which was heated to 60°C (140°F). A slight nitrogen purge was employed over the amine that was charged to prevent it from coming into contact with any air that might enter into the reactor during the reaction. The stearic acid was then charged, mixed and heated to 60°C (140°F) after which the dimethyl sulfate was charged in increments sufficient to maintain the temperature of the reaction mixture at 60-76°C (140-170°F). The reaction is exothermic and the end point of the reaction is indicated to some degree when an exotherm is no longer obtained. When the reaction was completed, the resultant mixture was allowed to cool. The foregoing method produced a reaction mixture which was analyzed by NMR analysis showing that the mixture obtained had the following components:

| | % |
|---|---|
| Quaternary Ammonium compound | 46 |
| Fatty Acid | 22 |
| Tertiary Amine and Amine Salt | 19 |
| Methyl Ester | 13 |

## Claims

1. A method of producing a mixture of quaternary ammonium compound, fatty acid, fatty acid ester, and tertiary amine salt by reacting in a solvent free system, a tertiary amine, quaternizing agent, and fatty acid each in an amount so that said mixture will contain at least a quaternary ammonium compound, fatty acid, fatty acid ester and tertiary amine salt wherein said tertiary amine has the formula: 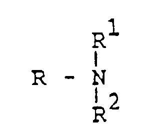 wherein R, R¹ and R² are:
(1) a linear or branched chain aliphatic saturated or unsaturated hydrocarbon group having up to 22 carbon atoms;
(2) a hydroxy lower alkyl group;
(3) an alkyl amide alkylene group of the formula; 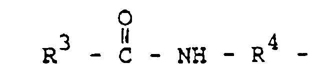 where R⁴ is lower alkylene and wherein R³ is (1), (2), (4) or (5);
(4) a lower alkoxy group; or
(5) a poly(oxyloweralkylene) group;
so that at least one of R, R¹, and R² is one of said hydrocarbon groups or one of said hydroxy lower alkyl groups;
or said tertiary amine is an imidazoline of the formula: 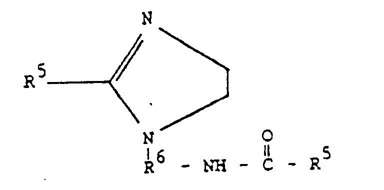 wherein R⁵ is a linear or branched chain aliphatic saturated or unsaturated hydrocarbon group having up to 22 carbon atoms and R⁶ is a lower alkylene group,
or an imidazoline having the formula: 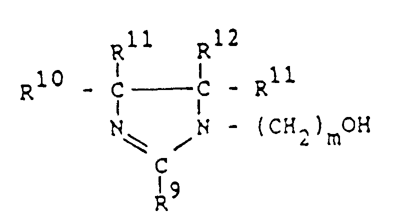 where R⁹ is alkyl or alkenyl having 8 to 30 carbon atoms;
R¹⁰ and R¹¹ are independently hydrogen or alkyl having up to 4 carbon atoms;
R¹² is alkyl having up to 4 carbon atoms;
m is 2 or 3;
said quaternizing agent being capable of producing a quaternary ammonium compound having anions A⁻;
said fatty acid is a linear or branched chain saturated or unsaturated fatty acid having from 12 to 22 carbon atoms;
said quaternary ammonium compound having the formula: 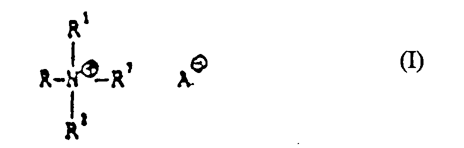 or 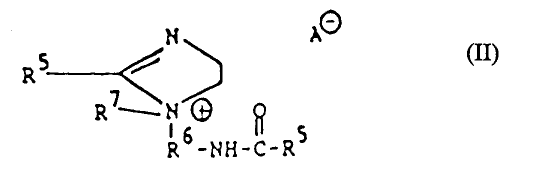 wherein R, R¹, R², R³, R⁴, R⁵ and R⁶ have the definition as described above, R⁷ is a lower alkyl or cyclo lower alkyl group and A⁻ is an anion; or 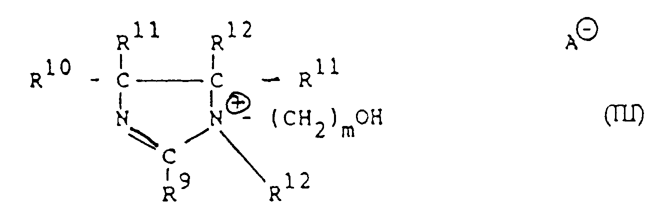 wherein R⁹, R¹⁰, R¹¹ and R¹² as well as m and A⁻ have the definition as described above;
said tertiary amine salt is a compound according to formula (I) or (II) or (III) wherein R⁷ or R¹² respectively is hydrogen;
said fatty acid ester having the formula 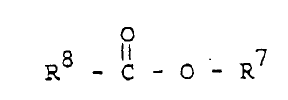 wherein R⁷ is a lower alkyl group or cyclo lower alkyl group and R⁸ a linear or branched chain, aliphatic saturated or unsaturated hydrocarbon group having from 11 to 21 carbon atoms and
where said tertiary amine, quaternizing agent and fatty acid are reacted in a single step reaction with one another in an amount so that the mixture obtained contains 10 wt.% to 80 wt.% of said quaternary ammonium compound, 15 wt.% to 70 wt.% of said salt, 1 wt.% to 70 wt.% of said ester and 5 wt.% to 70 wt.% of said fatty acid wherein the ratio of said quaternary ammonium compound to said salt is from 2:1 to 1:2 on a weight basis.

2. The method of claim 1 where said amine is of the formula: 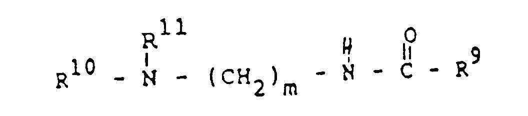 and said quaternary ammonium compound has the formula: 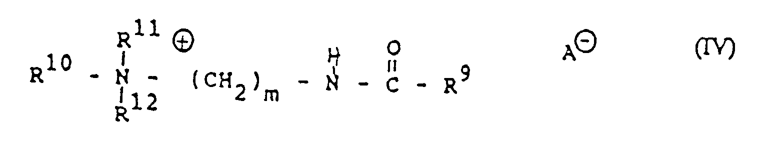 where R⁹ is alkyl or alkenyl having 8 to 22 carbon atoms;
R¹⁰ and R¹¹ are independently hydrogen or alkyl having up to 4 carbon atoms;
R¹² is alkyl having up to 4 carbon atoms;
m is 2 or 3;
A⁻ is an anion;
and the tertiary amine salt is a compound according to formula (IV) where R¹² on the nitrogen atom is hydrogen.

3. The method of claim 1 or 2 where said tertiary amine, quaternizing agent and fatty acid are reacted with one another in an amount so that said mixture obtained contains 35 wt.% to 55 wt.% of said quaternary ammonium compound, 10 wt.% to 30 wt.% of said salt, 5 wt.% to 25 wt.% of said ester and 15 wt.% to 35 wt.% of said fatty acid wherein the ratio of said quaternary ammonium compound to said salt is from 2:1 to 1:2 on a weight basis.

4. The method of claim 1 where said quaternary ammonium compound is (I) and wherein at least one of said R, R¹ and R² groups is a branched chain or linear, aliphatic saturated or unsaturated hydrocarbon group having from 12 to 22 carbon atoms and the balance, if any, of said R, R¹ and R² groups is a lower alkyl group.

5. The method of claim 4 where A is a lower alkyl sulfate group and where R, R¹ and R² are saturated hydrocarbon groups.

6. As a composition of matter, a solvent free mixture of
(i) a quaternary ammonium compound having the formula: 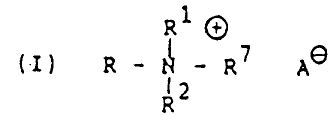 or 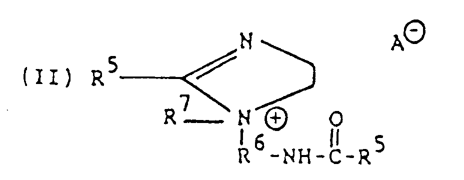 where R, R¹ and R² are
(1) linear or branched chain aliphatic saturated or unsaturated hydrocarbon group having up to 22 carbon atoms;
(2) a hydroxy lower alkyl group;
(3) an alkyl amido alkylene group of the formula, 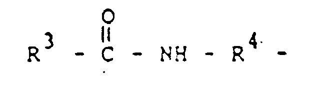
where R⁴ is lower alkylene and R³ is (1), (2), (4) or (5);
(4) a lower alkoxy group; or
(5) a poly(oxyloweralkylene) group;
so at least one of R, R¹, or R² is one of said hydrocarbon groups or one of said hydroxy lower alkyl groups
where R⁵ is a linear or branched chain aliphatic saturated or unsaturated hydrocarbon group having up to 22 carbon atoms, R⁶ is a lower alkylene group and R⁷ is a lower alkyl or cyclo lower alkyl group, or 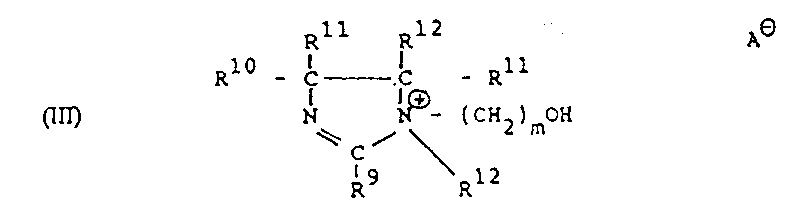 where R⁹ is alkyl or alkenyl having 8 to 30 carbon atoms;
R¹⁰ and R¹¹ are independently hydrogen or alkyl having up to 4 carbon atoms;
R¹² is alkyl having up to 4 carbon atoms;
m is 2 or 3;
where A⁻ is an anion;
(ii) a linear or branched chain saturated or unsaturated fatty acid having from 12 to 22 carbon atoms;
(iii) a tertiary amine salt having formula (I) or (II) or (III) where R⁷ or R¹² respectively is hydrogen and
(iv) a fatty acid ester having the formula: 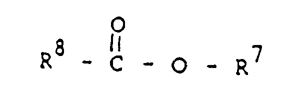 where R⁷ is a lower alkyl group or cyclo lower alkyl group and R⁸ is a linear or branched chain, aliphatic saturated or unsaturated group having from 11 to 21 carbon atoms and
where said components are present in an amount so that said mixture contains 10 wt. % of 80 wt. % of said quaternary ammonium compound, 15 wt. % to 70 wt. % of said salt, 1 wt. % to 70 wt. % of said ester and 5 wt. % to 70 wt. % of said fatty acid wherein the ratio of said quaternary ammonium compound to said salt is from about 2:1 to about 1:2 on a weight basis.

7. The composition of claim 6 where said quaternary ammonium compound is based on an amine having the formula: 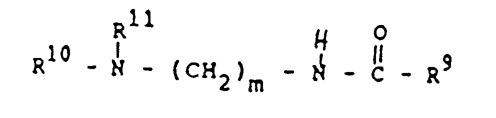 and said quaternary ammonium compound has the formula 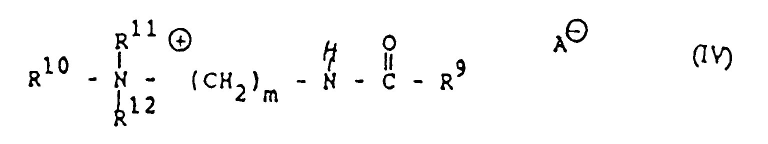 where R⁹ is alkyl or alkenyl having 8 to 22 carbon atoms;
R¹⁰ and R¹¹ are independently hydrogen or alkyl having up to 4 carbon atoms;
R¹² is alkyl having up to 4 carbon atoms;
m is 2 or 3;
A⁻ is an anion;
and the tertiary amine salt is a compound according to formula (IV) where R¹² on the nitrogen atom is hydrogen.

8. The composition of any of claims 6 or 7 where said components are present in an amount so that said mixture contains 35 wt. % to 55 wt. % of said quaternary ammonium compound, 10 wt. % to 30 wt. % of said salt, 5 wt. % to 25 wt. % of said ester and 15 wt. % to 35 wt. % of said fatty acid wherein the ratio of said quaternary ammonium compound to said salt is from 2:1 to 1:2 on a weight basis.

9. The mixture of claim 6 where said quaternary ammonium compound is (I) and wherein at least one of said R, R¹ and R² groups is a branched chain or linear, aliphatic saturated or unsaturated hydrocarbon group having from 12 to 22 carbon atoms and the balance, if any, of said R, R¹ and R² groups is a lower alkyl group.

10. The mixture of claim 8 or 9 where A⁻ is a lower alkyl sulfate group and where R, R¹ and R² are saturated hydrocarbon groups.

11. A fabric softening article of manufacture comprising a fabric softening amount of any of the mixtures of any of claims 6 to 10, operatively associated with a substrate that will release said mixture to a fabric under fabric softening conditions.

12. A fabric cleaning composition comprising a detergent in combination with a fabric softening amount of the mixtures of any of claims 6 to 10.

## Patentansprüche

1. Verfahren zur Herstellung von einer Mischung aus einer quaternären Ammoniumverbindung, einer Fettsäure, einem Fettsäureester und einem tertiären Aminsalz durch in einem lösungsmittelfreien System erfolgendes Umsetzen von einem tertiären Amin, einem Quaternisierungsmittel und einer Fettsäure in jeweils einer solchen Menge, daß die Mischung mindestens eine quaternäre Ammoniumverbindung, eine Fettsäure, einen Fettsäureester und ein tertiäres Aminsalz enthält,
wobei das tertiäre Amin die Formel 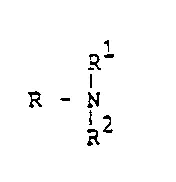 aufweist,
in welcher r, R¹ und R² bedeuten:
(1) eine lineare oder verzweigtkettige, aliphatische, gesättigte oder ungesättigte Kohlenwasserstoffgruppe mit bis zu 22 Kohlenstoffatomen,
(2) eine Hydroxyniederalkylgruppe,
(3) eine Alkylamidalkylengruppe gemäß der Formel 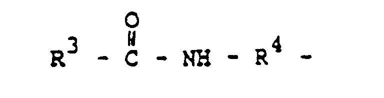
in welcher R⁴ ein Niederalkylen und R³ (1), (2), (4) oder (5) sind,
(4) eine niedere Alkoxygruppe oder
(5) eine Poly(oxy-niederalkylen)gruppe,
und wobei wenigstens ein R, R¹ und R² einer der Kohlenwasserstoffgruppen oder der Hydroxyniederalkylgruppen entspricht,
oder das tertiäre Amin ein Imidazolin gemäß der Formel 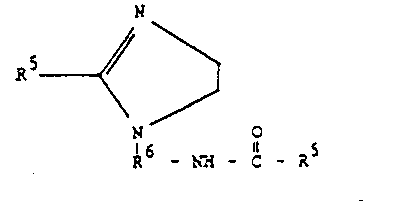 ist in welcher
R⁵ eine lineare oder verzweigtkettige, aliphatische, gesättigte oder ungesättigte Kohlenwasserstoffgruppe mit bis zu 22 Kohlenstoffatomen und R⁶ eine Niederalkylengruppe darstellen,
oder ein Imidazolin gemäß der Formel 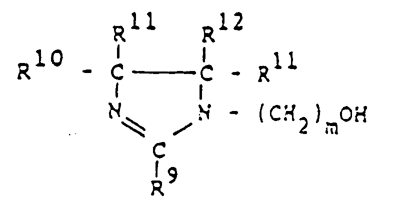 ist, in welcher
R⁹ einem Alkyl oder Alkenyl mit 8 bis 30 Kohlenstoffatomen entspricht,
R¹⁰ und R¹¹ unabhängig Wasserstoff oder Alkyl mit bis zu 4 Kohlenstoffatomen wiedergeben,
R¹² Alkyl mit bis zu 4 Kohlenstoffatomen bedeutet und m 2 oder 3 ist.
das Quaternisierungsmittel zur Bildung einer quaternären Ammoniumverbindung mit den Anionen A⁻ fähig ist,
die Fettsäure eine lineare oder verzweigtkettige, gesättigte oder ungesättigte Fettsäure mit 12 bis 22 Kohlenstoffatomen ist,
die quaternäre Ammoniumverbindung der Formel 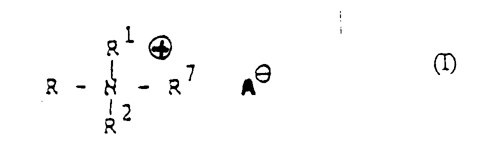 oder 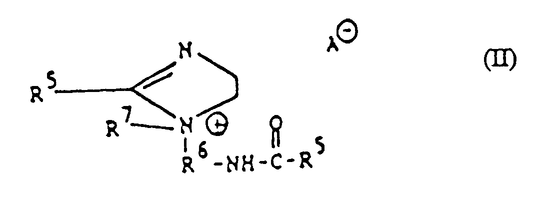 entspricht, wobei R, R¹, R², R³, R⁴, R⁵ und R⁶ die oben angeführte Bedeutung haben; R⁷ eine Niederalkyl- oder Cycloniederalkylgruppe ist und A⁻ ein Anion darstellt,
oder
die Formel 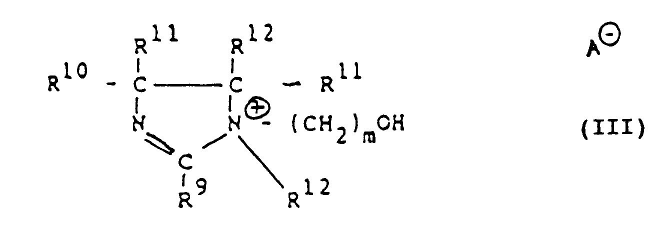 aufweist, in welcher R⁹, R¹⁰, R¹¹ und R¹² ebenso wie m und A⁻ der obigen Definition entsprechen,
das tertiäre Aminsalz eine Verbindung gemäß der Formel (I) oder (II) oder (III) ist, in welcher R⁷ oder R¹² Wasserstoff bedeuten,
der Fettsäureester die Formel 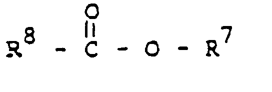
hat, in welcher R⁷ eine Niederalkylgruppe oder Cyclonniederalkylgruppe ist sowie R⁸ eine lineare oder verzeigtkettige, aliphatische, gesättigte oder ungesättigte Kohlenwasserstoffgruppe mit 11 bis 21 Kohlenstoffatomen darstellt,
und wobei das tertiäre Amin, das Quaternisierungsmittel und die Fettsäure in einstufiger Reaktion in einer solchen Menge miteinander umgesetzt werden, daß die resultierende Mischung 10 bis 80 Gew.-% von der quaternären Ammoniumverbindung, 15 bis 70 Gew.-% von dem Salz, 1 bis 70 Gew.-% von dem Ester und 5 bis 70 Gew.-% von der Fettsäure bei einem auf das Gewicht bezogenen Verhältnis von der quaternären Ammoniumverbindung zu dem Salz von 2 : 1 bis 1 : 2 enthält.

2. Verfahren gemäß Anspruch 1,
wobei das Amin der Formel 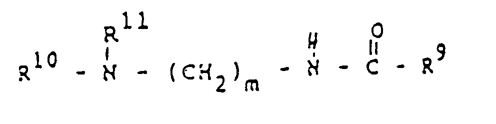 und die quaternäre Ammoniumverbindung der Formel 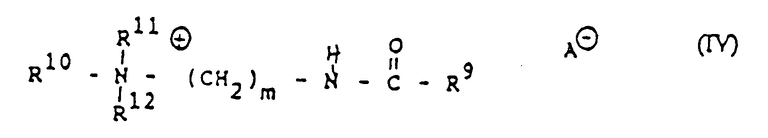 entsprechen, in denen
R⁹ ein Alkyl oder Alkenyl mit 8 bis 22 Kohlenstoffatomen ist,
R¹⁰ und R¹¹ unabhängig Wasserstoff oder Alkyl mit bis zu 4 Kohlenstoffatomen darstellen,
R¹² ein Alkyl mit bis zu 4 Kohlenstoffatomen wiedergibt, m 2 oder 3 ist,
A⁻ ein Anion bedeutet,
und das tertiäre Aminsalz eine Verbindung gemäß der Formel (IV) ist, in welcher R¹² am Stickstoffatom Wasserstoff bedeutet.

3. Verfahren gemäß Anspruch 1 oder 2,
wobei das tertiäre Amin, das Quaternisierungsmittel und die Fettsäure miteinander in einer solchen Menge umgesetzt werden, daß die resultierende Mischung 35 bis 55 Gew.-% von der quaternären Ammoniumverbindung, 10 bis 30 Gew.-% von dem Salz, 5 bis 25 Gew.-% von dem Ester und 15 bis 35 Gew.-% von der Fettsäure bei einem auf das Gewicht bezogenen Verhältnis von der quaternären Ammoniumverbindung zu dem Salz von 2 : 1 bis 1 : 2 enthält.

4. Verfahren gemäß Anspruch 1,
wobei die quaternäre Ammoniumverbindung (I) entspricht und wenigstens eine der R-, R¹- und R²-Gruppen eine verzweigtkettige oder lineare, aliphatische, gesättigte oder ungesättigte Kohlenwasserstoffgruppe mit 12 bis 22 Kohlenstoffatomen ist und eine jede der verbleibenden anderen R-, R¹- und R²-Gruppen eine niedere Alkylgruppe ist.

5. Verfahren gemäß Anspruch 4,
wobei A eine Niederalkylsulfatgruppe ist und R, R¹ und R² eine gesättigte Kohlenwasserstoffgruppe bedeuten.

6. Stoffzusammensetzung aus einer lösungsmittelfreien Mischung von
(i) einer quaternären Ammoniumverbindung gemäß der Formel 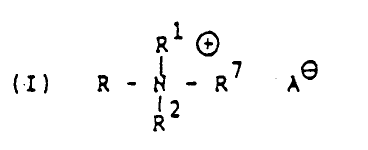 oder 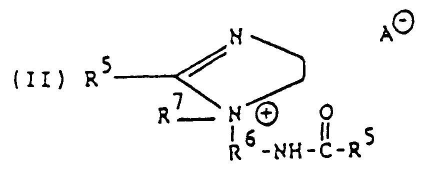 in denen R, R¹ und R² bedeuten:
(1) eine lineare oder verzweigtkettige, aliphatische, gesättigte oder ungesättigte Kohlenwasserstoffgruppe mit bis zu 22 Kohlensstoffatomen,
(2) eine Hydroxyniederalkylgruppe,
(3) eine Alkylamidalkylengruppe gemäß der Formel 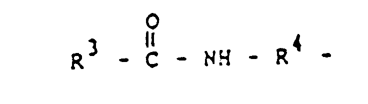 in welcher R⁴ ein Niederalkylen ist und R₃ (1), (2), (4) und (5) sind,
(4) eine niedere Alkoxygruppe oder
(5) eine Poly(oxy-niederalkylen)gruppe,
und wobei wenigstens ein R, R¹ und R² einer der Kohlenwasserstoffgruppen oder einer der Hydroxyniederalkylgruppen entspricht,
und R⁵ eine lineare oder verzweigtkettige, aliphatische, gesättigte oder ungesättigte Kohlenwasserstoffgruppe mit bis zu 22 C-Atomen wiedergibt,
R⁶ eine Niederalkylengruppe darstellt und
R⁷ eine Niederalkyl- oder Cycloniederalkyl-Gruppe ist,
oder der Formel 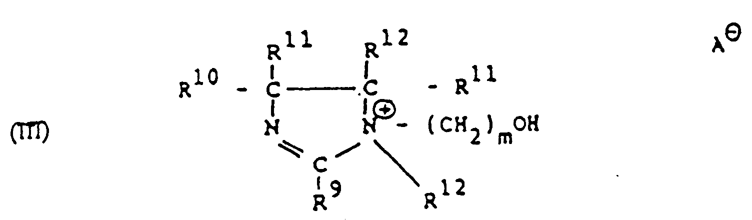 in der
R⁹ einem Alkyl oder Alkenyl mit 8 bis 30 Kohlenstoffatomen entspricht,
R¹⁰ und R¹¹ unabhängig Wasserstoff oder Alkyl mit bis zu 4 Kohlenstoffatomen wiedergeben,
R¹² Alkyl mit bis zu 4 Kohlenstoffatomen bedeutet,
m 2 oder 3 ist,
A⁻ ein Anion darstellt,
(ii) einer linearen oder verzweigtkettigen, gesättigten oder ungesättigten Fettsäure mit 12 bis 22 Kohlenstoffatomen,
(iii) einem tertiären Aminsalz gemäß einer der Formeln (I) oder (II) oder (III), wobei R⁷ oder R¹² jeweils Wasserstoff entsprechen,
(iv) einem Fettsäureester gemäß der Formel 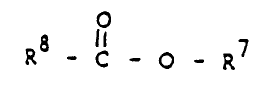 in der R⁷ eine Niederalkylgruppe oder Cycloniederalkylgruppe und R⁸ eine lineare oder verzweigtkettige, aliphatische, gesättigte oder ungesättigte Gruppe mit 11 bis 21 Kohlenstoffatomen darstellen,
und in der die vorgenannten Komponenten in solchen Mengen vorliegen, daß sie 10 bis 80 Gew.-% von der quaternären Ammoniumverbindung, 15 bis 70 Gew.-% von dem Salz, 1 bis 70 Gew.-% von dem Ester und 5 bis 70 Gew.-% von der Fettsäure bei einem auf das Gewicht bezogenen Verhältnis von der quaternären Ammoniumverbindung zu dem Salz von etwa 2 : 1 bis etwa 1 : 2 enthält.

7. Stoffmischung gemäß Anspruch 6,
in der die quaternäre Ammoniumverbindung auf einem Amin gemäß der Formel 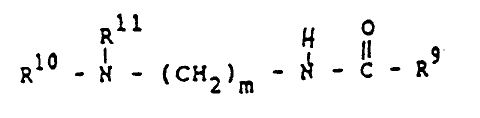 beruht
und die quaternäre Ammoniumverbindung die Formel 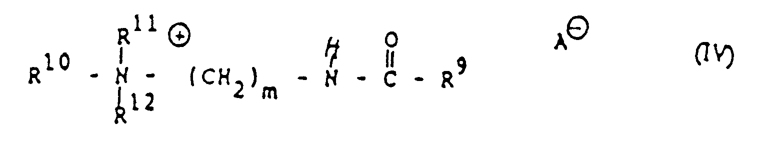 aufweist, in der
R⁹ ein Alkyl oder Alkenyl mit 8 bis 22 Kohlenstoffatomen ist,
R¹⁰ und R¹¹ unabhängig voneinander Wasserstoff oder Alkyl mit bis zu 4 Kohlenstoffatomen darstellen,
R¹² Alkyl mit bis zu 4 Kohlenstoffatomen entspricht,
m 2 oder 3 ist,
A⁻ ein Anion wiedergibt,
und das tertiäre Aminsalz eine Verbindung gemäß der Formel (IV) ist, worin R¹² am Stickstoffatom Wasserstoff bedeutet.

8. Stoffmischung gemäß einem der Ansprüche 6 oder 7,
wobei die Komponenten in einer solchen Menge vorliegen, daß sie 35 bis 55 Gew.-% von der quaternären Ammoniumverbindung, 10 bis 30 Gew.-% von dem Salz, 5 bis 25 Gew.-% von dem Ester und 15 bis 35 Gew.-% von der Fettsäure bei einem auf das Gewicht bezogenen Verhältnis von der quaternären Ammoniumverbindung zu dem Salz von 2 : 1 bis 1 : 2 enthält.

9. Mischung gemäß Anspruch 6,
wobei die quaternäre Ammoniumverbindung der Formel (I) entspricht und darin wenigstens einer der R-, R¹- und R²-Gruppen eine verzweigtkettige oder lineare, aliphatische, gesättigte oder ungesättigte Kohlenwasserstoffgruppe mit 12 bis 22 Kohlenstoffatomen ist und die restlichen anderen der R-, R¹- und R²- Gruppen Niederalkylgruppen sind.

10. Mischung gemäß Anspruch 8 oder 9,
wobei A⁻ eine Niederalkylsulfatgruppe ist und R, R¹ und R² gesättigte Kohlenwasserstoffgruppen sind.

11. Fertigungsgegenstand zur Gewebeweichmachung, enthaltend eine zum Gewebeweichmachen hinreichende Menge von einer der Mischungen gemäß einem jeden der Ansprüche 6 bis 10 auf einem funktionsgemäß zugeordneten Substrat, aus dem die Mischung unter den Bedingungen zum Gewebeweichmachen auf das Gewebe abgegeben wird.

12. Gewebereinigungsmittel, enthaltend ein Detergens in Kombination mit einer für das Gewebeweichmachen hinreichenden Menge an einer der Mischungen gemäß einem jeden der Ansprüche 6 bis 10.

## Revendications

1. Procédé de production d'un mélange d'un composé d'ammonium quaternaire, d'un acide gras, d'un ester d'un acide gras et d'un sel d'amine tertiaire, par la réaction d'un système sans solvant, d'une amine tertiaire, d'un agent de quaternisation et d'un acide gras, chacun en une quantité telle que ledit mélange contienne au moins un composé d'ammonium quaternaire, un acide gras, un ester d'un acide gras et un sel d'une amine tertiaire, où l'amine tertiaire a la formule suivante : 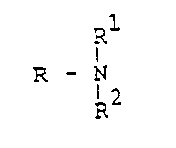 dans laquelle R, R¹ et R² représentent :
(1) un groupe hydrocarboné, saturé ou insaturé, aliphatique, à chaîne droite ou ramifiée, ayant jusqu'à 22 atomes de carbone ;
(2) un groupe hydroxy(alkyle inférieur) ;
(3) un groupe alkylamidealkylène de formule : 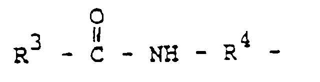 dans laquelle R⁴ est un radical alkylène inférieur, et R³ est (1), (2), (4) ou (5) ;
(4) un groupe alcoxy inférieur ; ou
(5) un groupe poly((oxy(alkylène inférieur)) ;
de façon qu'au moins l'un des radicaux R, R¹ et R² soit l'un desdits groupes hydrocarbonés ou l'un desdits groupes hydroxy(alkyle inférieur) ;
ou encore ladite amine tertiaire est une imidazoline de formule : 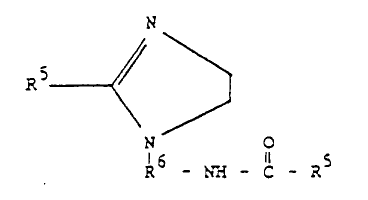 dans laquelle R⁵ est un groupe hydrocarboné saturé ou insaturé, aliphatique, à chaîne droite ou ramifiée, ayant jusqu'à 22 atomes de carbone, et R⁶ est un groupe alkylène inférieur,
ou une imidazoline ayant la formule : 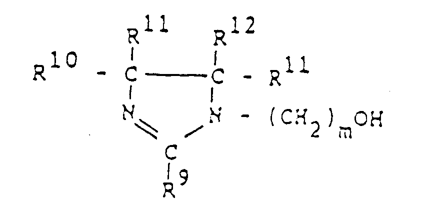 dans laquelle R⁹ est un radical alkyle ou alcényle ayant de 8 à 30 atomes de carbone ;
R¹⁰ et R¹¹, indépendamment l'un de l'autre, sont chacun un hydrogène ou un radical alkyle ayant jusqu'à 4 atomes de carbone ;
R¹² est un radical alkyle ayant jusqu'à 4 atomes de carbone ; m vaut 2 ou 3 ;
ledit agent de quaternisation étant capable de produire un composé d'ammonium quaternaire ayant des anions A⁻ ;
ledit acide gras est un acide gras saturé ou insaturé, à chaîne droite ou ramifiée, ayant de 12 à 22 atomes de carbone ;
ledit composé d'ammonium quaternaire ayant la formule suivante: 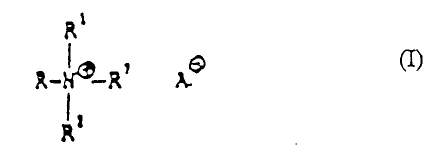 ou 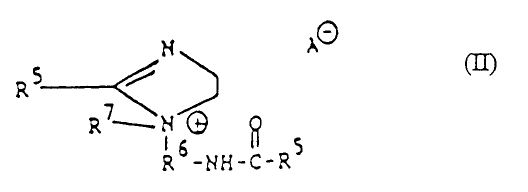 dans laquelle R, R¹, R², R³, R⁴, R⁵ et R⁶ ont les significations données ci-dessus, R⁷ est un groupe alkyle inférieur ou cycloalkyle inférieur et A- est un anion ; ou 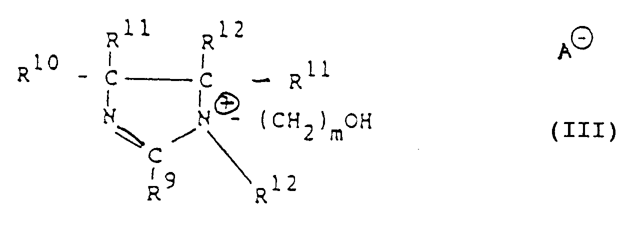 dans laquelle R⁹, R¹⁰, R¹¹ et R¹², ainsi que m et A-, ont les significations données ci-dessus ;
ledit sel d'amine tertiaire est un composé de formule (I) ou (II) ou (III) dans laquelle R⁷ ou R¹² est un hydrogène ;
ledit ester d'acide gras ayant la formule suivante : 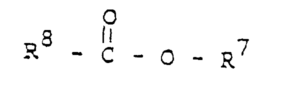 dans laquelle R⁷ est un groupe alkyle inférieur ou un groupe cycloalkyle inférieur, et R⁸ est un groupe hydrocarboné saturé ou insaturé, aliphatique, à chaîne droite ou ramifiée, ayant de 11 à 21 atomes de carbone, et
où ladite amine tertiaire, ledit agent de quaternisation et ledit acide gras sont mis à réagir dans le cadre d'une réaction en une seule étape les avec les autres, en une quantité telle que le mélange obtenu contienne de 10 à 80 % en poids dudit composé d'ammonium quaternaire, de 15 à 70 % en poids dudit sel, de 1 à 70 % en poids dudit ester et de 5 à 70 % en poids dudit acide gras, le rapport dudit composé d'ammonium quaternaire audit sel étant de 2:1 à 1:1 sur une base pondérale.

2. Procédé selon la revendication 1, dans lequel ladite amine a la formule suivante : 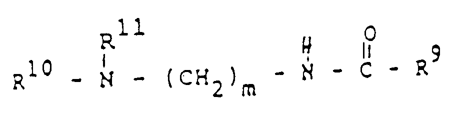 et ledit composé d'ammonium quaternaire a la formule suivante : 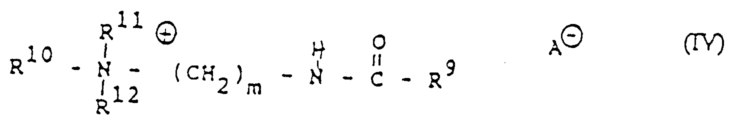 où R⁹ est un radical alkyle ou alcényle ayant de 8 à 22 atomes de carbone ;
R¹⁰ et R¹¹, indépendamment l'un de l'autre, sont chacun un hydrogène ou un radical alkyle ayant jusqu'à 4 atomes de carbone ;
R¹² est un radical alkyle ayant jusqu'à 4 atomes de carbone ;
n vaut 2 ou 3 ;
A- est un anion ;
et le sel d'amine tertiaire est un composé de formule (IV) dans laquelle R¹², sur l'atome d'azote, est un hydrogène.

3. Procédé selon la revendication 1 ou 2, dans lequel ladite amine tertiaire, ledit agent de quaternisation et ledit acide gras sont mis à réagir les uns avec les autres en une quantité telle que ledit mélange obtenu contienne de 35 à 55 % en poids dudit composé d'ammonium quaternaire, de 10 à 30 % en poids dudit sel, de 5 à 25 % en poids dudit ester, et de 15 à 35 % en poids dudit acide gras, le rapport dudit composé d'ammonium quaternaire audit sel étant de 2:1 à 1:2 sur une base pondérale.

4. Procédé selon la revendication 1, dans lequel ledit composé d'ammonium quaternaire est (I), et où au moins l'un desdits groupes R, R¹ et R² est un groupe hydrocarboné saturé ou insaturé, aliphatique, à chaîne droite ou ramifiée, ayant de 12 à 22 atomes de carbone, l'éventuel reste desdits groupes R, R¹ et R² représentant des groupes alkyle inférieur.

5. Procédé selon la revendication 4, dans lequel A est un groupe alkylsulfate inférieur, et R, R¹ et R² sont des groupes hydrocarbonés saturés.

6. Comme composition de matière, un mélange sans solvant
(i) d'un composé d'ammonium quaternaire ayant la formule suivante : 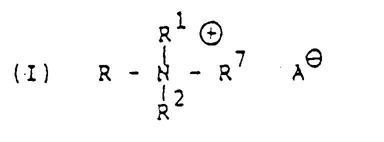 ou 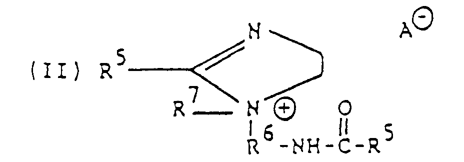 dans laquelle R, R¹ et R² représentent :
(1) un groupe hydrocarboné, saturé ou insaturé, aliphatique, à chaîne droite ou ramifiée, ayant jusqu'à 22 atomes de carbone ;
(2) un groupe hydroxy(alkyle inférieur) ;
(3) un groupe alkylamidealkylène de formule : 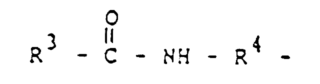 dans laquelle R⁴ est un radical alkylène inférieur, et R³ est (1), (2), (4) ou (5) ;
(4) un groupe alcoxy inférieur ; ou
(5) un groupe poly((oxy(alkylène inférieur)) ;
de façon qu'au moins l'un des radicaux R, R¹ et R² soit l'un desdits groupes hydrocarbonés ou l'un desdits groupes hydroxy(alkyle inférieur) ;
où R⁵ est un groupe hydrocarboné, saturé ou insaturé, aliphatique, à chaîne droite ou ramifiée, ayant jusqu'à 22 atomes de carbone, R⁶ est un groupe alkylène inférieur et R⁷ est un groupe alkyle inférieur ou cycloalkyle inférieur,
ou 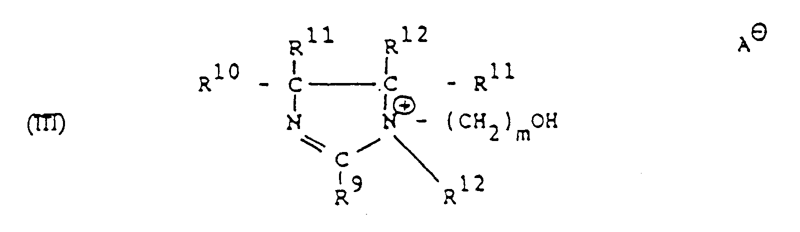 dans laquelle R⁹ est un radical alkyle ou alcényle ayant de 8 à 30 atomes de carbone ;
R¹⁰ et R¹¹, indépendamment l'un de l'autre, sont chacun un hydrogène ou un radical alkyle ayant jusqu'à 4 atomes de carbone ;
R¹² est un radical alkyle ayant jusqu'à 4 atomes de carbone ; m vaut 2 ou 3 ;
où A- est un anion ;
(ii) d'un acide gras, saturé ou insaturé, à chaîne droite ou ramifiée ayant de 12 à 22 atomes de carbone ;
(iii) d'un sel d'amine tertiaire ayant la formule (I) ou (II) ou (III) où R⁷ ou R¹² sont des hydrogènes et
(iv) d'un ester d'un acide gras ayant la formule suivante: 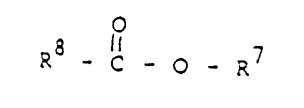 où R⁷ est un groupe alkyle inférieur ou un groupe cycloalkyle inférieur, et R⁸ est un groupe saturé ou insaturé, aliphatique, à chaîne droite ou ramifiée, ayant de 11 à 21 atomes de carbone, et
où lesdits constituants sont présents en une quantité telle que ledit mélange contienne de 10 à 80 % en poids dudit composé d'ammonium quaternaire, de 15 à 70 % en poids dudit sel, de 1 à 70 % en poids dudit ester et de 5 à 70 % en poids dudit acide gras, le rapport dudit composé d'ammonium quaternaire audit sel étant d'environ 2:1 à environ 1:2 sur une base pondérale.

7. Composition selon la revendication 6, dans laquelle ledit composé d'ammonium quaternaire est à base d'une amine ayant la formule suivante : 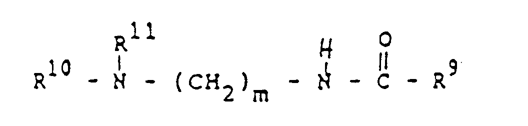 et ledit composé d'ammonium quaternaire a la formule suivante : 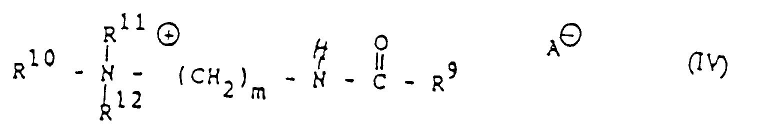 où R⁹ est un radical alkyle ou alcényle ayant de 8 à 22 atomes de carbone ;
R¹⁰ et R¹¹, indépendamment l'un de l'autre, sont chacun un hydrogène ou un radical alkyle ayant jusqu'à 4 atomes de carbone ;
R¹² est un radical alkyle ayant jusqu'à 4 atomes de carbone ;
m vaut 2 ou 3 ;
A- est un anion ;
et ledit sel d'amine tertiaire est un composé de formule (IV) dans laquelle R¹², sur l'atome d'azote, est un hydrogène.

8. Composition selon l'une quelconque des revendications 6 ou 7, dans laquelle lesdits constituants sont présents en une quantité telle que ledit mélange obtenu contienne de 35 à 55 % en poids dudit composé d'ammonium quaternaire, de 10 à 30 % en poids dudit sel, de 5 à 25 % en poids dudit ester, et de 15 à 35 % en poids dudit acide gras, le rapport dudit composé d'ammonium quaternaire audit sel étant de 2:1 à 1:2 sur une base pondérale.

9. Mélange selon la revendication 6, dans lequel ledit composé d'ammonium quaternaire est (I), et où au moins l'un desdits groupes R, R¹ et R² est un groupe hydrocarboné saturé ou insaturé, aliphatique, à chaîne droite ou ramifiée, ayant de 12 à 22 atomes de carbone, l'éventuel reste desdits groupes R, R¹ et R² représentant des groupes alkyle inférieur.

10. Mélange selon la revendication 8 ou 9, dans lequel A- est un groupe alkylsulfate inférieur, et R, R¹ et R² sont des groupes hydrocarbonés saturés.

11. Article de fabrication adoucissant des textiles, comprenant une quantité à effet adoucissant des textiles de l'un quelconque des mélanges selon l'une quelconque des revendications 6 à 10, en association opérationnelle avec un substrat qui va libérer ledit mélange dans un textile dans les conditions d'un adoucissement des textiles.

12. Composition de nettoyage des textiles comprenant un détergent en combinaison avec une quantité à effet adoucissant des textiles des mélanges selon l'une quelconque des revendications 6 à 10.
